# EUROPEAN PATENT APPLICATION

(11) **EP 4 706 613 A1**
(43) Date of publication of application: **11.03.2026**
(21) Application number: 25182262.3
(22) Date of filing: 12.06.2025
(51) Int. Cl.: A61F 13/15, A01K 1/015, A61F 5/48, B32B 3/26, D04H 1/732, B26F 1/00

(54) **APPARATUS AND METHOD FOR PRODUCING ABSORBENT BED PADS OR ABSORBENT PET MATS**

(30) Priority: 04.09.2024 IT 202400019678
(71) Applicant: GDM S.p.A., 40133 Bologna (IT)
(72) Inventor: ZAVALLONI, Alessandro, 40133 Bologna (IT)
(74) Representative: Porta & Consulenti Associati S.p.A.

(57) **Abstract**

The invention concerns an apparatus (100) for producing absorbent bed pads or absorbent pet mats, of the type comprising an absorbent core comprising a support layer and a plurality of absorbent fibres arranged on a first surface of said support layer. The apparatus (100) comprises a deposition station (120) comprising a conveyor belt (124) movable along an advancing direction (A) and configured to support a support layer strip (12), and a deposition device (122) configured to generate an air flow passing through the conveyor belt (124) and deposit said plurality of absorbent fibres onto a deposition surface of the support layer strip (12). The apparatus (100) also comprises a preparation station (110) arranged upstream of the deposition station (120) and of the conveyor belt (124) along said advancing direction (A) and comprising perforating members (114) configured to perforate the support layer strip (12) along its entire width.

The invention also concerns a method that can be carried out by said apparatus (100).

## Description

The present invention refers to an apparatus and a method for producing absorbent bed pads or absorbent pet mats.

Absorbent pads act as a barrier between a user and the bed or other surfaces on which the user stands. Similarly, the absorbent mats act as a barrier between the pet and the litter box or other surfaces on which the pet stands. In the following description reference will be made to the non-limiting example of absorbent bed pads, but what will be described also applies to the absorbent mats.

The absorbent bed pads are articles of assistance for the management of the incontinence. They are designed to absorb any leakage of urine or other body secretions during sleep, keeping the bed dry and comfortable.

Typically, an absorbent bed pad has a substantially rectangular shape and comprises a lower layer (also called "backsheet"), an upper layer (also called "topsheet") and an absorbent core arranged between the lower layer and the upper layer.

The lower layer is configured to be arranged on the bed and is made of a material impermeable to the body fluids, for example polyethylene or bioplastic.

The upper layer is configured to be arranged in a position contiguous to the user's body and is made of a material that is permeable to the body fluids. This layer is typically made of a soft, breathable material and allows the body fluid to pass through this layer and reach the absorbent core below, keeping the user dry and reducing the risk of irritation.

The absorbent core comprises a support layer and a plurality of absorbent fibres arranged on a surface of the support layer. The absorbent core has the function of absorbing the body fluids that pass through the upper layer and retaining them without leakage even for several hours.

The support layer is generally made of paper.

Typically, the absorbent fibres comprise cellulose and/or one or more super-absorbent polymers (SAP), for example sodium polyacrylate, typically in granular form, which may be confined inside, or within a coating of, cellulose or other similar materials.

The realization of the absorbent bed pads envisages, among other things, supplying a support layer strip onto a conveyor belt and depositing the absorbent fibres on a deposition surface of the support layer strip, over the entire width of the latter.

The deposition of the absorbent fibres takes place by fall from a deposition device arranged above the conveyor belt. The fall of the absorbent fibres is aided by an air flow which, coming from the deposition device, passes through the support layer strip and the conveyor belt.

The Applicant has found that the support layer strip generates a resistance to the passage of the air flow and, since the paper of which the support layer strip is typically formed has not a homogeneous structure, this resistance is not uniform along the width of the support layer strip. This causes an inhomogeneous distribution of the absorbent fibres on the support layer strip, to the detriment of the quality of the produced absorbent pads.

The Applicant has thought about how to overcome this drawback and has realised that it is possible to do so by perforating the support layer strip along its entire width before depositing the absorbent fibres on the support layer strip, thus making the resistance to the passage of the air flow substantially uniform along the entire width of the support layer strip.

The present invention therefore concerns, in a first aspect thereof, an apparatus for producing absorbent bed pads, or absorbent pet mats, of the type comprising an absorbent core comprising a support layer and a plurality of absorbent fibres arranged on a first surface of the support layer.

Preferably, a deposition station is provided.

Preferably, the deposition station comprises a conveyor belt.

Preferably, the conveyor belt is movable along an advancing direction.

Preferably, the conveyor belt is configured to support a support layer strip.

Preferably, a deposition device is provided.

Preferably, the deposition device is configured to deposit the plurality of absorbent fibres on a deposition surface of the support layer strip.

Preferably, the deposition device is configured to generate an air flow passing through the conveyor belt.

Preferably, a preparation station is provided.

Preferably, the preparation station is arranged upstream of the deposition station along the advancing direction.

Preferably, the preparation station is arranged upstream of the conveyor belt along the advancing direction.

Preferably, the preparation station comprises perforating members.

Preferably, the perforating members are configured to perforate the support layer strip along its entire width.

Thanks to the perforation carried out by the aforesaid perforating members the air flow coming from the deposition device during the deposition of the absorbent fibres on the support layer strip substantially uniformly passes through the support layer strip along its entire width. In this way it is possible to obtain a homogeneous distribution of the absorbent fibres on the support layer strip, to the benefit of the quality of the produced absorbent pads.

In a second aspect thereof, the present invention concerns a method for producing absorbent bed pads, or absorbent pet mats, of the type comprising an absorbent core comprising a support layer and a plurality of absorbent fibres arranged on a first surface of the support layer.

Preferably, a support layer strip is supplied towards a conveyor belt.

Preferably, the conveyor belt is movable along an advancing direction.

Preferably, the conveyor belt is configured to support the support layer strip.

Preferably, a plurality of absorbent fibres is deposited on a deposition surface of the support layer strip while the conveyor belt is passed through by an air flow.

Preferably, before the support layer strip reaches the conveyor belt, the support layer strip is perforated along its entire width.

The method of the invention can be carried out by employing an apparatus according to the first aspect of the present invention and allows to achieve the same advantages discussed with reference to the apparatus of the invention.

The present invention may have, in both aspects discussed above, at least one of the preferred features described below. These features can therefore be present individually or in combination with each other, unless expressly stated otherwise, both in the apparatus of the first aspect of the present invention and in the method of the second aspect of the present invention.

Preferably, the preparation station further comprises first folding members.

Preferably, the first folding members are configured to fold opposite longitudinal flaps of the support layer strip over a service surface of the support layer strip opposite the deposition surface.

Preferably, the first folding members are configured to continuously fold the opposite longitudinal flaps of the support layer strip over the service surface while the support layer strip passes through the preparation station.

Preferably, the first folding members are arranged at opposite sides with respect to the support layer strip when the support layer strip passes through the preparation station.

Preferably, the first folding members comprise first folding portions/elements arranged on one side with respect to the support layer strip and second folding portions/elements arranged on the opposite side with respect to the support layer strip.

The first and second folding portions/elements are preferably shaped and oriented in such a way as to interfere with the longitudinal flaps of the support layer strip during the movement of the support layer strip in the preparation station and fold them until reaching the service surface.

Preferably, the first folding members are movable along a transverse direction with respect to a first advancing path of the support layer strip in the preparation station. This movement makes it possible to realize absorbent bed pads of different sizes. In particular, it is possible to set the mutual distance between the first folding portions/elements and the second folding portions/elements as a function of the desired deposition surface width.

In a first preferred embodiment, the perforating members comprise a first roller configured to make a plurality of first holes on the entire support layer strip.

The first roller can be arranged upstream or downstream of the first folding members along the first advancing path.

Preferably, the first roller is arranged downstream of the first folding members along the first advancing path.

Preferably, the perforating members further comprise a pair of second rollers configured to each make a plurality of respective second holes at opposite longitudinal flaps of the support layer strip. The second rollers then make an additional perforation on the support layer strip only at the longitudinal flaps. This expedient is particularly advantageous in the case where the aforesaid longitudinal flaps are folded over before the deposition of the absorbent fibres on the support layer strip, as in a preferred embodiment of the present invention. In this case, in fact, the second rollers make an additional perforation on the support layer strip only at the longitudinal flaps folded over, that is where the support layer strip has two overlapping layers. This expedient makes it possible to uniform, along the entire width of the support layer strip, the resistance offered by this strip to the air flow coming from the deposition device during the deposition of the absorbent fibres in the case where the support layer strip has its end flaps folded over, contributing to the achievement of a more homogeneous distribution of the absorbent fibres on the support layer strip.

Preferably, the pair of second rollers is arranged downstream of the first folding members along the first advancing path.

The pair of second rollers can be arranged upstream or downstream of the first roller along the first advancing path.

Preferably, the second rollers are movable along a transverse direction with respect to the first advancing path, so that they can be arranged at the longitudinal flaps folded over of the support layer strip when the support layer strip passes through the preparation station.

In a second preferred embodiment, the perforating members comprise a roller comprising a central portion configured to make a plurality of first holes on a central longitudinal portion of the support layer strip.

Preferably, this roller further comprises opposite end portions configured to each make a plurality of respective second holes at opposite longitudinal flaps of the support layer strip.

Preferably, the aforesaid roller is arranged downstream of the first folding members along the first advancing path.

Preferably, the plurality of respective second holes differs from the plurality of first holes in at least one of the following parameters: size of the holes and number of holes for the same area.

Preferably, the deposition station comprises a pair of air blowers arranged downstream of the deposition device, at opposite sides with respect to the advancing direction.

The air blowers perform an air jet cut that allows to clearly separate the absorbent fibres deposited over the deposition surface from the others (here referred to as excess fibres), for the benefit of the quality of the produced absorbent pads.

Preferably, the air blowers are movable along a transverse direction orthogonal to the advancing direction, so as to be able to make absorbent bed pads of different sizes.

Preferably, the air blowers are arranged upstream of a downstream end portion of the conveyor belt along the advancing direction. The excess fibres are then separated from the others and recovered before the support layer strip leaves the conveyor belt.

Preferably, prior to depositing the plurality of absorbent fibres onto the deposition surface of the support layer strip, opposite longitudinal flaps of the support layer strip are folded over a service surface opposite the deposition surface.

This expedient allows to separate with precision, by means of the aforesaid air blowers, the excess absorbent fibres from those deposited on the deposition surface of the support layer strip in the case where it is wished to make absorbent pads provided with folds at the opposite longitudinal edges. In this case, in fact, during this separation the concentrated air jets emitted by the blowers do not impact on the longitudinal flaps of the support layer strip since they have been previously folded over the service surface.

In a first preferred embodiment, perforating the support layer strip comprises making a plurality of first holes on the entire support layer strip.

Preferably, perforating the support layer strip further comprises, before making or after having made the plurality of first holes, making a plurality of second holes at opposite longitudinal flaps of the support layer strip.

In a second preferred embodiment, perforating the support layer strip comprises making a plurality of first holes on a central longitudinal portion of the support layer strip.

Preferably, perforating the support layer strip further comprises making, simultaneously with the first holes, a plurality of second holes at opposite longitudinal flaps of the support layer strip.

Preferably, in both aforesaid preferred embodiments, the plurality of second holes differs from the plurality of first holes in at least one of the following parameters: size of the holes and number of holes for the same area.

Further characteristics and advantages of the present invention will become clearer from the following detailed description of preferred embodiments thereof, made with reference to the appended drawings and provided by way of indicative and non-limiting example, in which:
- figure 1 is a schematic cross-sectional view of a support layer strip used to produce absorbent bed pads by an apparatus in accordance with the present invention, such as that of figure 7, this support layer strip being in an initial operating configuration thereof;
- figure 2 is a schematic cross-sectional view of the support layer strip of figure 1 in a second intermediate operating configuration thereof;
- figure 3 is a schematic cross-sectional view of a portion of the apparatus of figure 7 with the support layer strip in the intermediate operating configuration;
- figure 4 is a schematic cross-sectional view of the support layer strip brought back to its initial operating configuration after absorbent fibres have been deposited thereon;
- figure 5 is a schematic cross-sectional view of the support layer strip of figure 1 in a final operating configuration thereof after the absorbent fibres have been deposited thereon;
- figure 6 is a schematic cross-sectional view of an absorbent bed pad comprising the support layer strip in its final operating configuration;
- figure 7 is a schematic side elevational view of an apparatus in accordance with the present invention;
- figure 8 is a schematic side elevation view on an enlarged scale of a first embodiment of a station of the apparatus of figure 7;
- figure 9 is a schematic plan view of some members present in the station of figure 8;
- figure 10 is a schematic plan view of a portion of the support layer strip when it is located at the plane X of figure 8;
- figure 11 is a schematic plan view of a portion of the support layer strip when it is located at the plane XI of figure 8;
- figure 12 is a schematic side elevation view on an enlarged scale of a second embodiment of the station of the apparatus of figure 7, alternative to that of figure 8;
- figure 13 is a schematic plan view of a member present in the station of figure 12;
- figure 14 is a schematic plan view of a portion of the support layer strip when it is located at the plane XIV of figure 12;
- figure 15 is a schematic perspective view on an enlarged scale of a further member of the apparatus of figure 7.

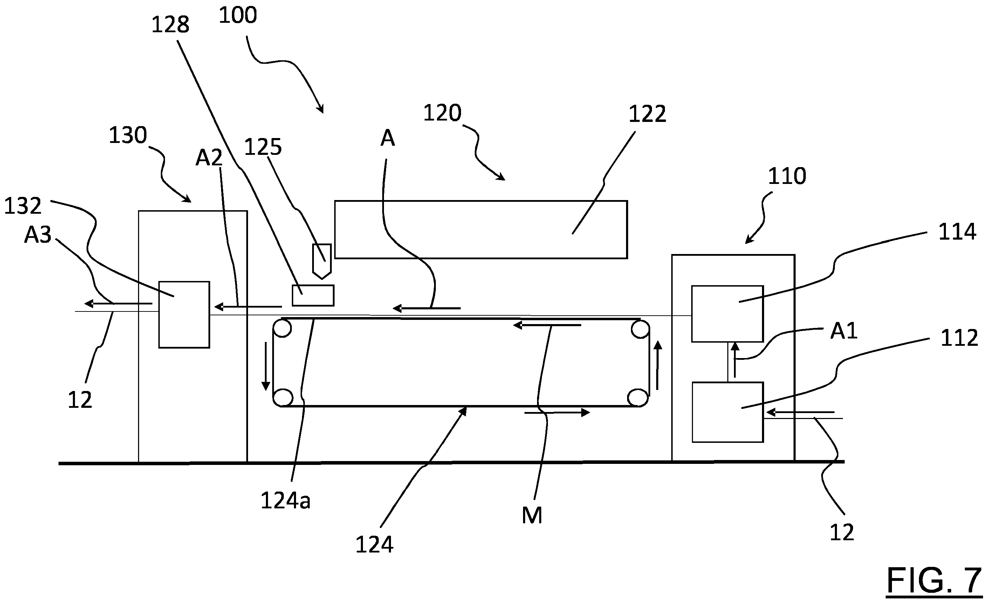

In figure 7, the reference numeral 100 is used to indicate an apparatus for producing absorbent bed pads in accordance with the present invention.

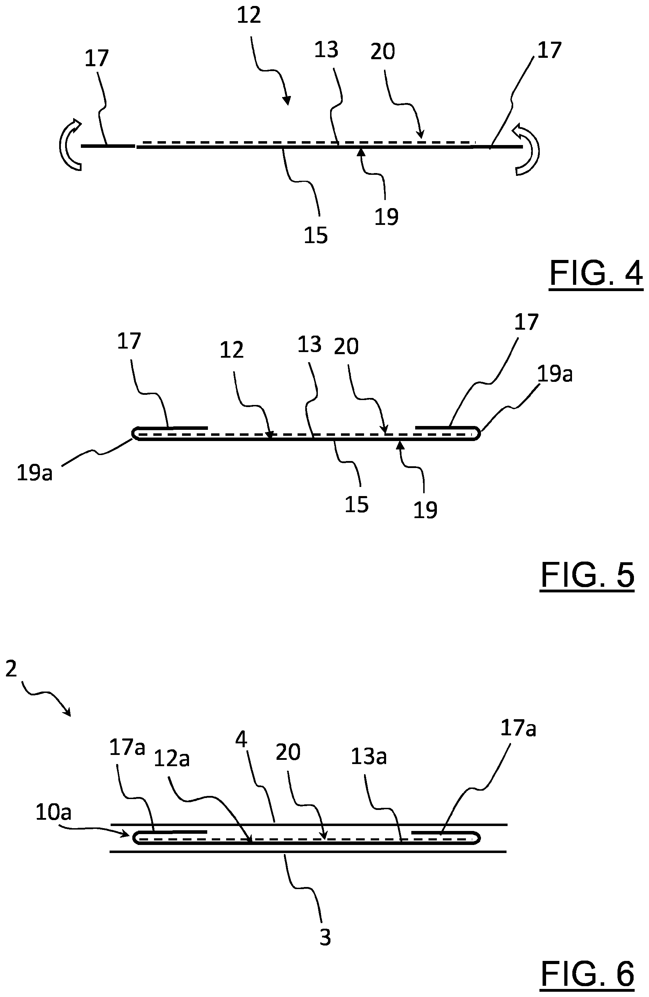

Without for this reason losing in generality, explicit reference will be made below to an absorbent pad, such as the one illustrated in figure 6 and indicated with reference numeral 2. In figure 6 the absorbent pad 2 is voluntarily illustrated with the relative components spaced apart from each other, so as to be able to clearly distinguish them. In reality these components are in contact with each other.

The absorbent pad 2 has a substantially rectangular shape and comprises a lower layer 3 (also called "backsheet"), an upper layer 4 (also called "topsheet") superimposed on the lower layer 3 and an absorbent core 10a arranged between the lower layer 3 and the upper layer 4.

In the following of the present description and in the following claims, the terms "upper", "lower", "above", "below" and the like are used referring to the position assumed by the absorbent pad 2 when it is arranged on a bed (not illustrated), with the lower layer 3 in contact with the bed and the upper layer 4 arranged in such a way as to come into contact with the body of the user.

The lower layer 3 is made of a material impermeable to the body fluids, for example polyethylene, polypropylene or bioplastic, while the upper layer 4 is made of a material permeable to the body fluids, for example non-woven fabric.

The absorbent core 10a comprises a support layer 12a and a plurality of absorbent fibres 20 arranged on a surface 13a of the support layer 12a. The surface 13a faces the upper layer 4.

The support layer 12a may for example be made of paper, while the absorbent fibres 20 comprise cellulose and/or one or more super-absorbent polymers (SAP), for example sodium polyacrylate, typically in granular form, which may be confined inside, or within a coating of, cellulose or other similar materials.

The support layer 12a has opposite side flaps 17a folded over the surface 13a, so that some absorbent fibres 20 are trapped between the surface 13a and the respective side flaps 17a.

The lower layer 3 and the upper layer 4 have a width substantially equal and greater than the width of the absorbent core 10a.

The absorbent pad 2 is produced starting from a support layer strip 12 by the apparatus of figure 7.

The support layer strip 12 has a deposition surface 13 destined to receive the absorbent fibres 20 and a service surface 15 opposite the deposition surface 13.

The apparatus 100 comprises a preparation station 110, a deposition station 120 arranged downstream of the preparation station 110 along an advancing direction A of the support layer strip 12 and a forming station 130 arranged downstream of the deposition station 120 along the advancing direction A.

In accordance with the present invention, the support layer strip 12 is initially supplied to the preparation station 110, from which it is then supplied to the deposition station 120, from which it is then supplied to the forming station 130.

In particular, the support layer strip 12 is supplied to the preparation station 110 with its opposite longitudinal flaps 17 arranged on the same plane of a central longitudinal portion 19 of the support layer strip 12, i.e. with the longitudinal flaps 17 arranged in what in this description is indicated as "initial position".

The support layer strip 12 is then supplied from the preparation station 110 to the deposition station 120 and from the latter to the forming station 130 with its opposite longitudinal flaps 17 folded over the service surface 15, i.e. with the longitudinal flaps 17 arranged in what in this description is indicated as "intermediate position".

The support layer strip 12 subsequently exits the forming station 130 with its opposite longitudinal flaps 17 folded over the deposition surface 13, i.e. with the longitudinal flaps 17 arranged in what in this description is indicated as the "final position".

The preparation station 110 comprises folding members 112 configured to continuously fold the opposite longitudinal flaps 17 of the support layer strip 12 over the service surface 15 while the support layer strip 12 passes through the preparation station 110 along an advancing path A1.

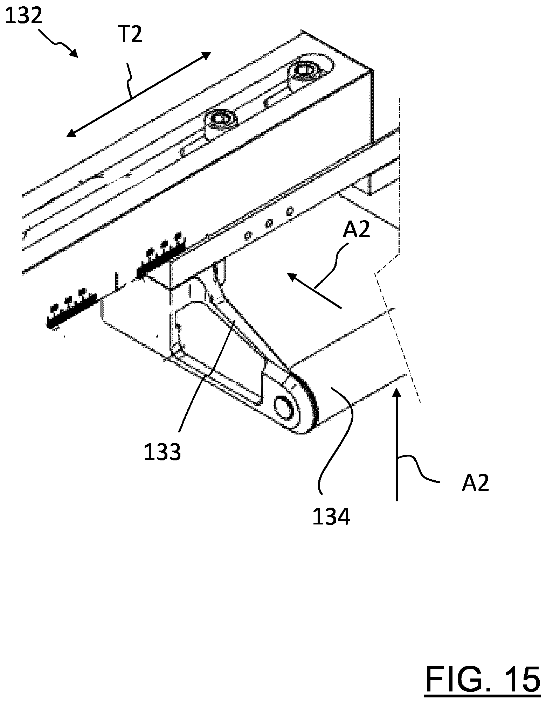

The folding members 112 may be of conventional type. For example, they may comprise suitably shaped and oriented folding elements, similar to those illustrated in figure 15 and described below.

The folding members 112 are movable along a transverse direction T with respect to the advancing path A1, so as to be able to be arranged from time to time in the desired position.

The preparation station 110 further comprises perforating members 114 configured to perforate the support layer strip 12 along its entire width while the support layer strip 12 passes through the preparation station 110 along the advancing path A1.

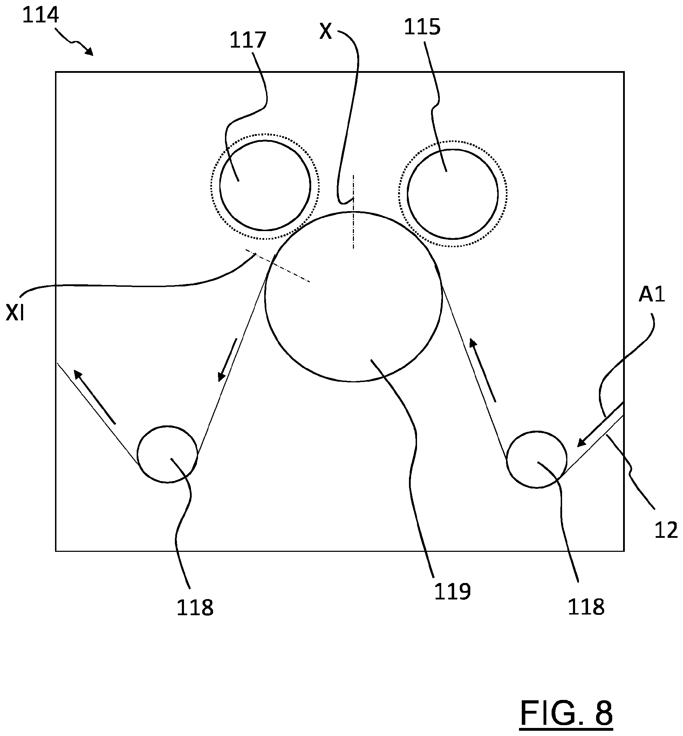

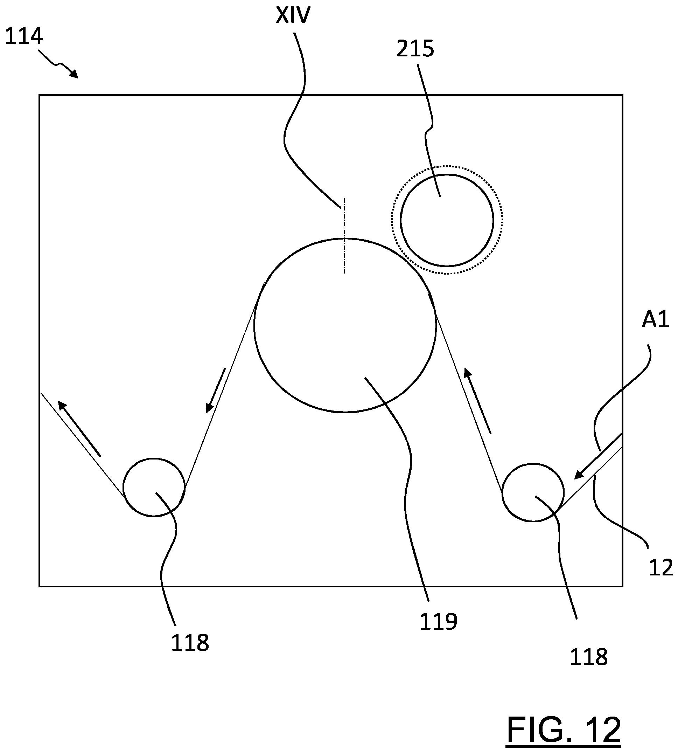

Two alternative embodiments of the perforating members 114 are illustrated in figures 8 and 12.

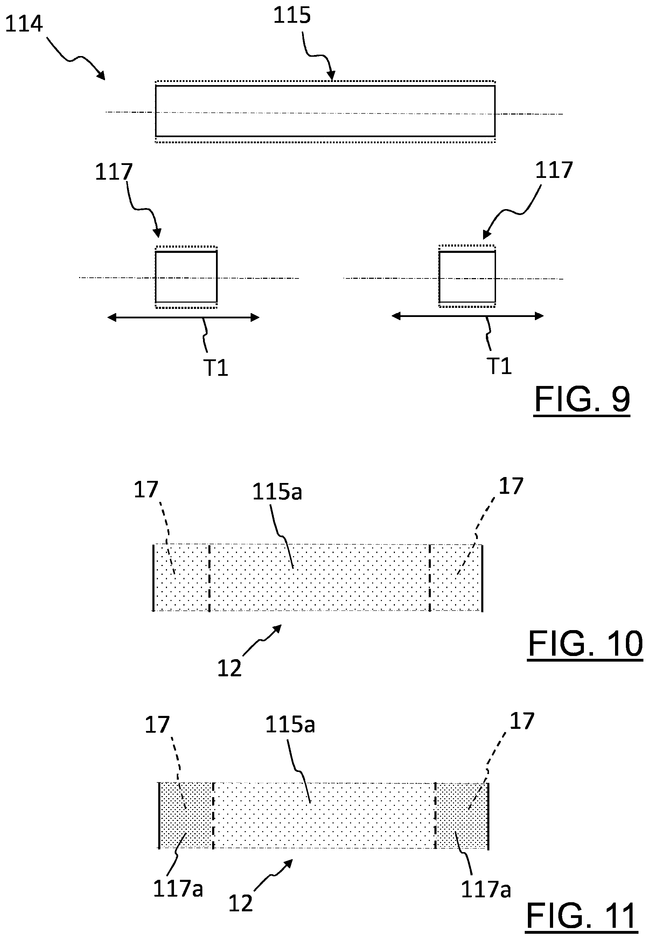

In the embodiment illustrated in figures 8 and 9, the perforating members 114 comprise a first roller 115 configured to make a plurality of first holes 115a on the entire support layer strip 12.

The first roller 115 is arranged downstream of the folding members 112 along the advancing path A1.

The perforating members 114 further comprise a pair of second rollers 117 configured to each make a plurality of respective second holes 117a at a respective longitudinal flap 17 of the support layer strip 12 after this longitudinal flap 17 has been folded over the service surface 15.

The pair of second rollers 117 is arranged downstream of the folding members 112 along the advancing path A1.

In the non-limiting example of figures 8 and 9, the second rollers 117 are aligned and arranged downstream of the first roller 115 along the advancing path A1. Alternatively, the second rollers 117 could be arranged upstream of the first roller 115 along the advancing path A1.

The second rollers 117 are movable along a transverse direction T1 orthogonal to the advancing path A1 so as to be able to be arranged at the longitudinal flaps 17.

The first roller 115 and the second rollers 117 are substantially tangent to a counter roller 119 on which the support layer strip 12 is arranged during its movement along the advancing path A1 and at which the first and second holes 115a, 117a are made.

The support layer strip 12 is tensioned on the counter roller 119 through a pair of return rollers 118, located upstream and downstream of the counter roller 119 along the advancing path A1.

Figure 10 is a view of a portion of the support layer strip 12 taken downstream of the first roller 115 along the advancing path A1, in which the first holes 115a are visible on the entire support layer strip 12.

Figure 11 is a view of a portion of the support layer strip 12 taken downstream of the second rollers 117 along the advancing path A1, in which also the second holes 117a are visible at the longitudinal flaps 17.

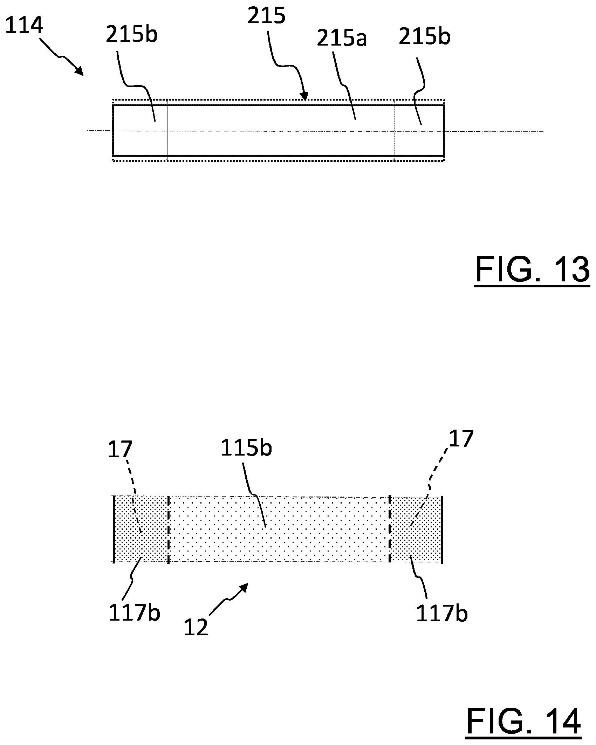

The embodiment illustrated in figures 12 and 13 differs from that illustrated in figures 8 and 9 only in that the perforating members 114 in this case comprise a single roller 215, in turn comprising a central portion 215a configured to make a plurality of first holes 115b on the central longitudinal portion 19 of the support layer strip 12 and opposite end portions 215b configured to each make a plurality of respective second holes 117b at a respective longitudinal flap 17 of the support layer strip 12 after this longitudinal flap 17 has been folded over the service surface 15.

The roller 215 is arranged downstream of the folding members 112 along the advancing path A1.

Figure 14 is a view of a portion of the support layer strip 12 taken downstream of the roller 215 along the advancing path A1, in which the first holes 115b and the second holes 117b are visible.

Preferably, in both embodiments of the perforating members 114 described above, the plurality of second holes 117a, 117b differs from the plurality of first holes 115a, 115b in at least one of the following parameters: size of the holes and number of holes for the same area.

In particular, the second holes 117a, 117b define at the longitudinal flaps 17 folded over the service surface 15 a density of air passage openings greater than that at the portion of support layer strip 12 in which only the first holes 115a, 115b are made.

The deposition station 120 comprises a deposition device 122 configured to deposit the absorbent fibres 20 on the deposition surface 13.

Thus, when the support layer strip 12 passes through the deposition station 120, the deposition surface 13 faces the deposition device 122.

The deposition station 120 also comprises a conveyor belt 124 configured to support the support layer strip 12 and supply it along the advancing direction A. The conveyor belt 124 has a width greater than that of the support layer strip 12 and is arranged below the deposition device 122. Further, the conveyor belt 124 is perforated. In figure 7 the support layer strip 12 is voluntarily illustrated raised with respect to the conveyor belt 124 to clearly illustrate the support layer strip 12 above the conveyor belt 124. In reality, the support layer strip 12 rests on the conveyor belt 124.

The deposition device 122 generates an air flow that facilitates the deposition of the absorbent fibres 20 on the deposition surface 13. The air flow passes through the support layer strip 12 and the conveyor belt 124.

The deposition of the absorbent fibres 20 therefore takes place by fall from the deposition device 122 and by the thrust action exerted by the aforesaid air flow.

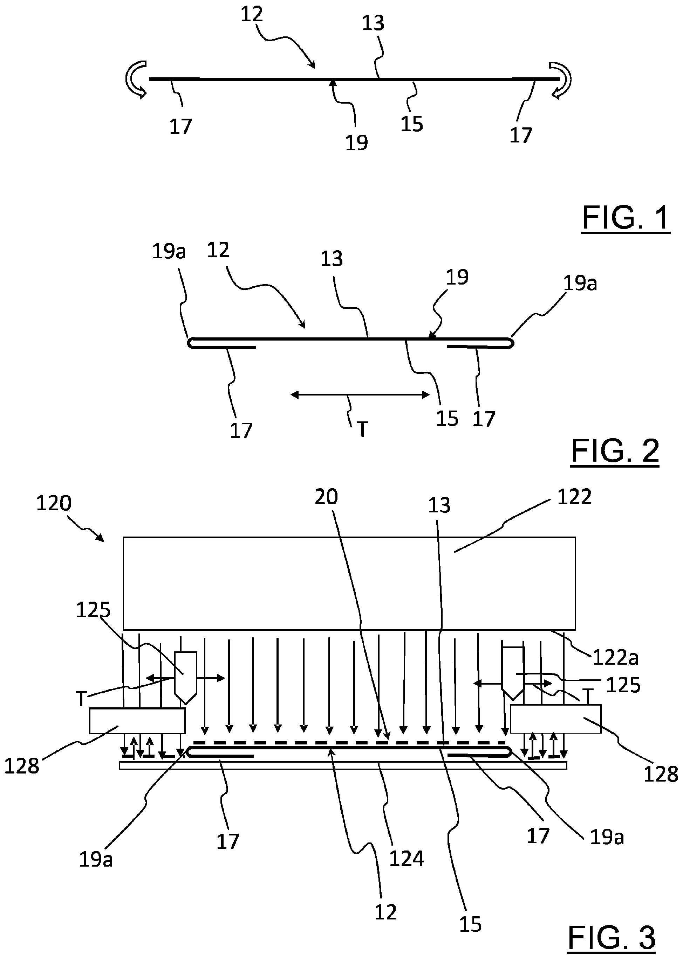

The absorbent fibres pass through an opening 122a defined in a lower surface of the deposition device 122. This opening 122a preferably has a width greater than that of the support layer strip 12. As shown in figure 3, the absorbent fibres 20 that do not fall onto the support layer strip 12 (herein referred to as "excess fibres") are sucked in by two suction nozzles 128 arranged above the conveyor belt 124 at opposite sides with respect to the latter.

The absorbent fibres 20 are deposited on the central longitudinal portion 19 of the support layer strip 12. The central longitudinal portion 19 has opposite longitudinal edges 19a, at which the longitudinal flaps 17 are folded over (figures 2 and 5). Thus, in the deposition station 120 the deposition surface 13 corresponds to the upper surface of the central longitudinal portion 19 of the support layer strip 12.

Two air blowers 125 are arranged downstream of the deposition device 122, with reference to the advancing direction A and at a downstream end portion 124a of the conveyor belt 124 with reference to a movement direction M thereof. The two air blowers 125 are positioned along a transverse direction with respect to the movement direction M, each near a respective side of the conveyor belt 124. Each air blower 125 is configured to generate a concentrated jet of air that laps the opposite longitudinal edges 19a and allows to clearly and precisely separate the excess absorbent fibres 20, i.e. those that following the fall are at least partially in an external position with respect to these longitudinal edges 19a, from the absorbent fibres 20 that instead are entirely deposited on the central longitudinal portion 19, and therefore on the deposition surface 13.

The air blowers 125 are movable along the transverse direction T orthogonal to the advancing direction A so as to be able to be arranged at the longitudinal edges 19a.

The forming station 130 comprises folding members 132 configured to continuously fold the opposite longitudinal flaps 17 of the support layer strip 12 over the deposition surface 13 while the support layer strip 12 passes through the forming station 130 along an advancing path A2.

The folding members 132 may be of conventional type.

By way of non-limiting example, the folding members 132 may be of the type illustrated in figure 15. They comprise folding elements 133 (figure 15 shows only one folding element 133) intended to come into contact with the longitudinal flaps 17 of the support layer strip 12 and fold these longitudinal flaps 17 over the deposition surface 13 while the support layer strip 12 passes through the forming station 130 along the advancing path A2 and after the support layer strip 12, supported by a counter roller 134 located upstream of the folding elements 133 along the advancing path A2, is oriented so as to interfere with the folding elements 133.

The folding members 132 are movable along a transverse direction T2 with respect to the advancing path A2 so as to be able to be arranged from time to time in the desired position.

Figures 4 and 5 show two operating configurations of the support layer strip 12 assumed in sequence in the forming station 130, and then after the absorbent fibres 20 have been deposited on the deposition surface 13.

The operating configuration of figure 4 is assumed after the folding members 132 have brought back the longitudinal flaps 17 on the same plane of the central longitudinal portion 19, i.e. into their initial position, illustrated in figure 1.

The operating configuration of figure 5 is assumed after the folding members 132 have folded the longitudinal flaps 17 over the central longitudinal portion 19, i.e. in their final position. In this final position the longitudinal flaps 17 cover the absorbent fibres 20 deposited near the opposite longitudinal edges 19a.

The apparatus 100 further comprises, downstream of the forming station 130 along an advancing direction A3 of the support layer strip 12 (which is in the operating configuration of figure 5), an application station (not illustrated because of conventional type) configured to apply the strip of lower layer 3 and the strip of upper layer 4 below and above the support layer strip 12, respectively, after the longitudinal flaps 17 have been folded over the deposition surface 13.

Obviously, a person skilled in the art, in order to satisfy specific and contingent needs, can make numerous modifications and variations to the invention described above while remaining within the scope of protection defined by the following claims.

## Claims

1. Apparatus (100) for producing absorbent bed pads, or absorbent pet mats, of the type comprising an absorbent core (10a) comprising a support layer (12a) and a plurality of absorbent fibres (20) arranged on a first surface (13a) of said support layer (12a), said apparatus (100) comprising:
- a deposition station (120) comprising a conveyor belt (124), movable along an advancing direction (A) and configured to support a support layer strip (12), and a deposition device (122), configured to generate an air flow passing through the conveyor belt (124) and deposit said plurality of absorbent fibres (20) onto a deposition surface (13) of the support layer strip (12);
- a preparation station (110) arranged upstream of the deposition station (120) and of the conveyor belt (124) along said advancing direction (A) and comprising perforating members (114) configured to perforate the support layer strip (12) along its entire width.

2. Apparatus (100) according to claim 1, wherein said preparation station (110) further comprises first folding members (112) configured to fold opposite longitudinal flaps (17) of the support layer strip (12) over a service surface (15) of the support layer strip (12) opposite said deposition surface (13).

3. Apparatus (100) according to claim 1 or 2, wherein said perforating members (114) comprise:
- a first roller (115) configured to make a plurality of first holes (115a) on the entire support layer strip (12);
- a pair of second rollers (117) each configured to make a plurality of respective second holes (117a) at opposite longitudinal flaps (17) of the support layer strip (12).

4. Apparatus (100) according to claim 3, wherein said second rollers (117) are movable along a transverse direction (T) with respect to an advancing path of the support layer strip (12) in the preparation station (110).

5. Apparatus (100) according to claim 1 or 2, wherein said perforating members (114) comprise a roller (215) comprising a central portion (215a) configured to make a plurality of first holes (115b) on a central longitudinal portion of the support layer strip (12) and opposite end portions (215b) each configured to make a plurality of respective second holes (117b) at opposite longitudinal flaps (17) of the support layer strip (12).

6. Apparatus (100) according to any one of claims 3 to 5, wherein said plurality of respective second holes (117a; 117b) differs from said plurality of first holes (115a; 115b) in at least one of the following parameters: size of holes and number of holes for the same area.

7. Method for producing absorbent bed pads, or absorbent pet mats, of the type comprising an absorbent core (10a) comprising a support layer (12a) and a plurality of absorbent fibres (20) arranged on a first surface (13a) of said support layer (12a), said method comprising:
- supplying a support layer strip (12) towards a conveyor belt (124) movable along an advancing direction (A) and configured to support the support layer strip (12);
- depositing a plurality of absorbent fibres (20) on a deposition surface (13) of the support layer strip (12) while an air flow passes through the conveyor belt (124);
- before the support layer strip (12) reaches the conveyor belt (124), perforating the support layer strip (12) along its entire width.

8. Method according to claim 7, further comprising, prior to depositing said plurality of absorbent fibres (20) on said deposition surface (13), folding opposite longitudinal flaps (17) of the support layer strip (12) over a service surface (15) opposite said deposition surface (13).

9. Method according to claim 7 or 8, wherein perforating said support layer strip (12) comprises:
- making a plurality of first holes (115a) over all the support layer strip (12);
- before or after making the plurality of first holes (115a), making a plurality of second holes (117a) at opposite longitudinal flaps (17) of the support layer strip (12).

10. Method according to claim 7 or 8, wherein perforating said support layer strip (12) comprises:
- making a plurality of first holes (115b) on a central longitudinal portion of the support layer strip (12);
- making, simultaneously with the first holes (115b), a plurality of second holes (117b) at opposite longitudinal flaps (17) of the support layer strip (12).

11. Method according to claim 9 or claim 10, wherein said plurality of second holes (117a; 117b) differs from said plurality of first holes (115a; 115b) in at least one of the following parameters: size of holes and number of holes for the same area.
